# EUROPÄISCHE PATENTANMELDUNG

(11) **EP 2 730 655 A1**
(43) Veröffentlichungstag der Anmeldung: **14.05.2014**
(21) Anmeldenummer: 12007663.3
(22) Anmeldetag: 12.11.2012
(51) Int. Cl.: C12N 15/52, C12N 9/02, C12N 9/18, C12P 7/62, C12P 7/64

(54) **Verfahren zur Umsetzung eines Carbonsäureesters unter Verwendung BioH-defizienter Zellen**

(71) Anmelder: Evonik Industries AG, 45128 Essen (DE)
(72) Erfinder: Schaffer, Steffen, Dr., 45699 Herten (DE); Andrea, Heiko, 45768 Marl (DE); Wessel, Mirja, Dr., 44799 Bochum (DE); Hennemann, Hans-Georg, Dr., 45770 Marl (DE); Häger, Harald, Dr., 59348 Lüdinghausen (DE)

(57) **Zusammenfassung**

Die Erfindung betrifft ein Verfahren zur Umsetzung eines Carbonsäureesters der Formel (I)

R¹-A-COOR² (I),

wobei R¹ aus der Gruppe ausgewählt ist, die Wasserstoff, -CH₂OH, -CHO, -COOR³, -CH₂SH, -CH₂OR³ und -CH₂NH₂ umfasst,
wobei R² aus der Gruppe ausgewählt ist, die Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst,
und wobei es sich bei A um einen Alkylrest mit wenigstens vier Kohlenstoffatomen handelt,
mittels einer Zelle, umfassend den Schritt
a) Kontaktieren der Zelle mit dem Carbonsäureester in einer wässrigen Lösung,

wobei es sich bei der Zelle um eine rekombinante Zelle handelt, bei der die Aktivität eines Polypeptides umfassend SEQ ID NO 2 oder eine Variante davon gegenüber dem Wildtyp der Zelle verringert ist, sowie eine dazu geeignete Zelle und deren Verwendungen.

SEQUENCE LISTING

<110>  Evonik Industries AG

<120>  Verfahren zur Umsetzung eines Carbonsäureesters

<130>  201200172

<160>  2

<170>  PatentIn version 3.5

<210>  1
<211>  771
<212>  DNA
<213>  Escherichia coli

<400>  1
atgaataaca tctggtggca gaccaaaggt caggggaatg ttcatcttgt gctgctgcac      60

ggatggggac tgaatgccga agtgtggcgt tgcattgacg aggaacttag ctcgcatttt     120

acgctgcacc ttgttgacct gcccggcttc gggcgtagcc ggggatttgg tgcgctgtca     180

cttgctgata tggccgaagc cgtgctgcaa caggcacctg ataaagccat ttggttaggc     240

tggagtctgg gcgggctggt ggcaagccag attgcgttaa cccatcccga gcgtgttcag     300

gcgctggtca ccgtggcgtc gtcaccttgt tttagtgctc gtgacgagtg gccggggata     360

aaaccggacg tgctggcggg atttcagcag caactcagtg atgattttca gcgtacagtg     420

gagcggttcc tggcgttaca aaccatgggg actgaaacgg cgcgccagga tgcgcgggcg     480

ttgaagaaaa ccgttctggc gttaccgatg ccggaggttg acgtgcttaa tggcgggctg     540

gaaatcctga aaacggtcga tctccgtcag ccgctgcaaa acgtgtccat gccgtttttg     600

cgattgtatg gctatctcga cggtctggtg ccgcgcaaag tggtgccgat gctggataaa     660

ctttggcctc acagcgaatc atatatcttc gccaaagcgg cccatgcgcc atttatttcg     720

catccggccg agttttgtca cctgctggtg gcgttgaagc agagggtgta g              771

<210>  2
<211>  256
<212>  PRT
<213>  Escherichia coli

<400>  2

Met Asn Asn Ile Trp Trp Gln Thr Lys Gly Gln Gly Asn Val His Leu
1               5                   10                  15

Val Leu Leu His Gly Trp Gly Leu Asn Ala Glu Val Trp Arg Cys Ile
            20                  25                  30

Asp Glu Glu Leu Ser Ser His Phe Thr Leu His Leu Val Asp Leu Pro
        35                  40                  45

Gly Phe Gly Arg Ser Arg Gly Phe Gly Ala Leu Ser Leu Ala Asp Met
    50                  55                  60

Ala Glu Ala Val Leu Gln Gln Ala Pro Asp Lys Ala Ile Trp Leu Gly
65                  70                  75                  80

Trp Ser Leu Gly Gly Leu Val Ala Ser Gln Ile Ala Leu Thr His Pro
                85                  90                  95

Glu Arg Val Gln Ala Leu Val Thr Val Ala Ser Ser Pro Cys Phe Ser
            100                 105                 110

Ala Arg Asp Glu Trp Pro Gly Ile Lys Pro Asp Val Leu Ala Gly Phe
        115                 120                 125

Gln Gln Gln Leu Ser Asp Asp Phe Gln Arg Thr Val Glu Arg Phe Leu
    130                 135                 140

Ala Leu Gln Thr Met Gly Thr Glu Thr Ala Arg Gln Asp Ala Arg Ala
145                 150                 155                 160

Leu Lys Lys Thr Val Leu Ala Leu Pro Met Pro Glu Val Asp Val Leu
                165                 170                 175

Asn Gly Gly Leu Glu Ile Leu Lys Thr Val Asp Leu Arg Gln Pro Leu
            180                 185                 190

Gln Asn Val Ser Met Pro Phe Leu Arg Leu Tyr Gly Tyr Leu Asp Gly
        195                 200                 205

Leu Val Pro Arg Lys Val Val Pro Met Leu Asp Lys Leu Trp Pro His
    210                 215                 220

Ser Glu Ser Tyr Ile Phe Ala Lys Ala Ala His Ala Pro Phe Ile Ser
225                 230                 235                 240

His Pro Ala Glu Phe Cys His Leu Leu Val Ala Leu Lys Gln Arg Val
                245                 250                 255

## Beschreibung

Die Erfindung betrifft ein Verfahren zur Umsetzung eines Carbonsäureesters der Formel (I)

R¹ - A - COOR² (I),

wobei R¹ aus der Gruppe ausgewählt ist, die Wasserstoff, -CH₂OH, -CHO, -COOR³, -CH₂SH, - CH₂OR³ und -CH₂NH₂ umfasst,
wobei R² aus der Gruppe ausgewählt ist, die Wasserstoff und Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst,
und wobei es sich bei A um einen Alkylrest mit wenigstens vier Kohlenstoffatomen handelt,
mittels einer Zelle, umfassend den Schritt
a) Kontaktieren der Zelle mit dem Carbonsäureester in einer wässrigen Lösung,
wobei es sich bei der Zelle um eine rekombinante Zelle handelt, bei der die Aktivität eines Polypeptides mit der SEQ ID NO 2 oder einer Variante davon gegenüber dem Wildtyp der Zelle verringert ist, sowie eine dazu geeignete Zelle und deren Verwendungen.

Die Biotechnologie beschäftigt sich mit der Herstellung u. a. von Feinchemikalien unter Einsatz von verschiedenen Organismen, die interessante Synthesefähigkeiten besitzen. Gegenüber herkömmlichen chemischen Verfahren weisen biotechnologische Verfahren eine Reihe von Vorteilen auf. Zunächst verzichten sie in der Regel komplett auf gesundheitsgefährende Stoffe wie schwermetallbasierte Katalysatoren und Reaktionsbedingungen mit extremen pH-, Druckund Temperaturwerten. Weiterhin kommt man beim Aufbau der für das biotechnologische Verfahren erforderlichen Infrastruktur vielfach mit geringeren Investitionen und Sicherheitsmaßnahmen aus. Die Selektivität und Spezifität von biotechnologisch relevanten Enzymen übersteigt die von chemischen Katalysatoren oft erheblich, so dass die Bildung unerwünschter und vom Produkt häufig schlecht abtrennbarer Nebenprodukte vermindert werden kann, die bei einer Synthese unter Verwendung synthetisch-organischer Verfahren notwendigerweise entstehen würden. Schließlich akzeptieren biotechnologisch relevante Organismen als Edukte in vielen Fällen Verbindungen wie komplexe Kohlenhydrate, die aus nachwachsenden Rohstoffen gewonnen werden können. Die Abhängigkeit eines produzierenden Unternehmens von fossilen Rohstoffen wie Erdöl und Erdgas kann somit verringert werden.

Die Etablierung biotechnologischer Verfahren ist jedoch mit erheblichen Schwierigkeiten verbunden, die dazu führen, dass heutzutage nur sehr wenige Stoffe im Industriemaßstab biotechnologisch hergestellt werden. Das Hauptproblem besteht darin, dass eine Zelle mit einer gewünschten Syntheseaktivität nicht nur das eine, für diese Syntheseaktivität verantwortliche Enzym aufweist, sondern vielmehr tausende Enzyme, die in der gleichen Zelle koexistieren und mit einander um Substrate konkurrieren oder gar komplett gegenläufige Reaktionen katalysieren. So sind allein im Genom von *Escherichia coli* ca. 80 mit bioinformatischen Verfahren als Hydrolasen identifizierte Polypeptide kodiert, d. h. Enzyme, die bestimmte Bindungen unter Verbrauch eines Wassermoleküls spalten. Unter welchen Bedingungen ein Enzym von der Zelle hergestellt werden und welche Reaktionen mit welchen Substraten es katalysiert, ist tatsächlich nur in wenigen Fällen erschöpfend geklärt. Die gezielte Auswahl eines Enzyms zur Katalyse einer bestimmten Reaktion ist damit in vielen Fällen nicht möglich.

Dementsprechend besteht beim Einsatz von Zellen als Biokatalysatoren statt chemischer oder isolierter biologischer Katalysatoren auch immer die Gefahr, dass ein von einem mit einer erwünschten Aktivität ausgestatteten Enzym hergestelltes Produkt oder Zwischenprodukt oder bereits das ursprüngliche Edukt von einem anderen Enzym in ein unerwünschtes Nebenprodukt umgewandelt wird. Ob dies geschieht und welches der zahlreichen Enzyme in diesem Fall die unerwünschte Aktivität aufweist, lässt sich trotz technischer Fortschritte auf dem Gebiet der Bioinformatik nicht vorhersehen.

Gerade bei chemisch reaktiven Stoffen, die industriell als reaktionsfähige Edukte für die Herstellung komplexerer Produkte begehrt sind, ist es nicht unwahrscheinlich, dass sie im Inneren der Zelle mit essentiellen Bestandteilen des Organismus reagieren und somit toxisch wirken. Ist das der Fall, so ist die Wachstums- und Synthesefähigkeit des Organismus bis hin zum Absterben der Zelle beeinträchtigt, ohne dass der Entwickler die Toxizität unmittelbar erkennen könnte. Welcher Organismus welche Konzentration an einem chemisch reaktiven Stoff verträgt, ist ebenfalls nicht vorhersehbar.

Bei Verfahren mit mehreren, jeweils von einem Enzym katalysierten Reaktionen erschwert die Komplexität des Systems die Suche nach Ausbeute oder Reinheit limitierenden Faktoren. Ist die Ausbeute an Produkt zu niedrig, so kann dies daran liegen, dass eines der Enzyme in einer zu niedrigen Konzentration vorhanden ist, ohne dass bekannt wäre, um welches der in Frage kommenden Enzyme es sich dabei handelt, d. h. das Edukt wird im vorgesehenen Zeitrahmen oder vor dem Abbau durch konkurrierende Enzyme aufgrund unzureichender Synthesekapazität nicht umgesetzt. Alternativ ist es durchaus möglich, dass ein Enzym zwar nachweisbar in der Zelle in Form eines Polypeptids vorhanden ist, aber gerade in dieser Zelle nicht die für die Aktivität essentielle Faltung aufweist oder ein bis dato unbekannter, für die Aktivität aber essentieller Cofaktor fehlt. Gleichermaßen kann, wie bereits erwähnt, das Stoffwechselprodukt für die Zelle toxisch sein oder abgebaut werden.

Der Fachmann, der ein biotechnologisches Verfahren etablieren oder verbessern möchte, sieht sich also mit zahlreichen möglichen Ansatzpunkten konfrontiert, erhält aus dem Stand der Technik in den meisten Fällen aber keine konkrete und umsetzbare Anweisung, an welchem dieser Ansatzpunkte er ansetzten muss, um zum Ziel zu gelangen.

Carbonsäureester stellen eine Gruppe industriell stark nachgefragter Verbindungen dar, die entweder selbst oder in Form von weiterverarbeiteten Produkten als Pharmazeutika, Kosmetika, Kunststoffe und dergleichen Verwendung finden.

Häufig wird für eine Verarbeitung jedoch nicht nur eine zunächst in ein Vorprodukt einzuführende Esterfunktion benötigt, sondern es muss am Ester eine weitere Derivatisierung durchgeführt werden, ohne dass die Esterfunktion dabei oder in der Folge hydrolysiert wird. Letzteres ist nicht trivial zu bewerkstelligen, da viele Carbonsäureester insbesondere in wässrigen Lösungen und bei vom Neutralpunkt stark abweichenden pH-Werten selbst in Abwesenheit derartige Reaktionen katalysierender Enzyme schon zur Hydrolyse neigen.

Eine wichtige Möglichkeit, Carbonsäureester weiter zu derivatisieren, besteht in der Oxidation darin enthaltener Alkylketten. Dabei entsteht zunächst ein Alkohol, der entweder als solcher eingesetzt oder zum Aldehyd oder Keton weiteroxidiert werden kann. Das Aldehyd oder Keton kann entweder reduktiv aminiert werden oder zur Carbonsäure weiteroxidiert werden, die bei Bedarf ihrerseits wieder verestert werden kann.

Diese vielfältigen Umsetzungsmöglichkeiten, von denen viele durch endogene, d. h. natürlich in einem Organismus vorhandene Enzyme katalysiert werden, zeigen, dass das Problem der ungesteuerten Nebenproduktbildung oder Verstoffwechselung bei der Umsetzung von Carbonsäureestern mittels biotechnologischer Verfahren besonders schwer wiegt.

Ein Beispiel für einen industriell stark nachgefragten Carbonsäureester, der herkömmlich ausgehend von im Erdöl enthaltenen Kohlenwasserstoffen hergestellt wird, ist 12-Aminolaurinsäuremethylester (ALSME). ALSME ist ein wichtiges Ausgangsprodukt bei der Herstellung von Polymeren, beispielsweise zur Herstellung von Leitungssystemen auf Nylonbasis. Bislang wird ALSME in einem Prozess mit niedriger Ausbeute ausgehend von fossilen Rohstoffen hergestellt.

Ein vielversprechender neuer Weg zur biotechnologischen Herstellung von ALS bzw. ALSME ist in der WO 2009/077461 beschrieben. Dabei wird Laurinsäuremethylester in einem ersten Schritt von einer Monooxygenase oxidiert, und das entstehende Aldehyd wird mittels einer Transaminase zum ALSME umgesetzt. Ein Nachteil dieses Verfahrens besteht darin, dass Nebenprodukte, beispielsweise die Dicarbonsäure, entstehen, die vom dem erwünschten Produkt ALSME nur unter Schwierigkeiten abgetrennt werden können. Das vermindert die Ausbeute und erschwert das Recycling von hydrophoben Lösungsmitteln und hydrophoben flüssigen Kationenaustauschern, die gemäß der PCT/EP2011/071491 zur Abtrennung des Produktes aus der wässrigen Reaktionsmischung verwendet werden können, auf Kosten der Effizienz bei der Ressourcennutzung.

Vor diesem Hintergrund besteht die der Erfindung zu Grunde liegende Aufgabe darin, ein mit Hinblick auf Ausbeute, Kohlenstoff- und/oder Stickstoffbilanz und/oder Reinheit möglichst effizientes biotechnologisches Verfahren zur Umsetzung von Carbonsäureestern bereitzustellen.

Eine weitere der Erfindung zu Grunde liegende Aufgabe besteht darin, ein mit Hinblick auf Ausbeute, Kohlenstoff- und/oder Stickstoffbilanz, Wiederverwendbarkeit verwendeter Agenzien und/oder Reinheit des Produktes möglichst effizientes biotechnologisches Verfahren zur Umsetzung von Carbonsäureestern zu aminierten Carbonsäureestern bereitzustellen. In diesem Zusammenhang wird unter eine effizienten Kohlenstoff- und/oder Stickstoffbilanz bevorzugt verstanden, dass sich ein möglichst hoher Anteil des zur Umsetzung eines Carbonsäureesters in Form von geeigneten Substraten an eine Zelle verfütterten Kohlenstoffs und/oder Stickstoffs im erwünschten Endprodukt wiederfindet, anstatt beispielsweise zu anderen Produkten als den erwünschten umgesetzt zu werden.

Eine weitere der Erfindung zu Grunde liegende Aufgabe besteht darin, die Aufarbeitbarkeit einer mehrphasigen Reaktionsmischung aus der Umsetzung eines Carbonsäureesters zu verbessern, besonders mit Hinblick auf die Wiederverwendbarkeit zur Aufarbeitung verwendeter hydrophober Lösungsmittel und flüssiger Kationenaustauscher, sowie mit Hinblick auf die Phasenbildung und -trennung bei einem biphasischen System umfassend eine wässrige Phase, in der die Umsetzung des Carbonsäureesters abläuft, und eine organische Phase mit organischen Lösungsmitteln und/oder flüssigen Kationenaustauschem.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

In einem ersten Aspekt wird das der Erfindung zu Grunde liegende Problem gelöst durch ein Verfahren zur Umsetzung eines Carbonsäureesters der Formel (I)

R¹ - A - COOR² (I),

wobei R¹ aus der Gruppe ausgewählt ist, die Wasserstoff, -CH₂OH, -CHO, -COOR³, -CH₂SH, - CH₂OR³ und -CH₂NH₂ umfasst,
wobei R² aus der Gruppe ausgewählt ist, die Wasserstoff und Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst,
und wobei es sich bei A um einen Alkylrest mit wenigstens vier Kohlenstoffatomen handelt,
mittels einer Zelle, umfassend den Schritt
a) Kontaktieren der Zelle mit dem Carbonsäureester in einer wässrigen Lösung,
wobei es sich bei der Zelle um eine rekombinante Zelle handelt, bei der die Aktivität eines Polypeptides umfassend SEQ ID NO 2 oder eine Variante davon gegenüber dem Wildtyp der Zelle verringert ist.

In einer ersten Ausführungsform des ersten Aspekts wird das Problem gelöst durch ein Verfahren, weiter umfassend den Schritt
b) Kontaktieren der wässrigen Lösung mit einer hydrophoben organischen Lösung umfassend einen Kationenaustauscher.

In einem zweiten Aspekt wird das der Erfindung zu Grunde liegende Problem gelöst durch eine Verwendung eines Knockouts eines für ein Polypeptid umfassend SEQ ID NO 2 oder eine Variante davon kodierenden Gens als Teil der genetischen Ausstattung einer rekombinanten Zelle zur Erhöhung der Produktion eines Carbonsäureesters der Formel (I)

R¹ - A - COOR² (I),

wobei R¹ aus der Gruppe ausgewählt ist, die Wasserstoff, -CH₂OH, -CHO, -COOR³, -CH₂SH, - CH₂OR³ und -CH₂NH₂ umfasst,
wobei R² aus der Gruppe ausgewählt ist, die Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst,
und wobei es sich bei A um einen Alkylrest mit wenigstens vier Kohlenstoffatomen handelt,
gegenüber dem entsprechenden Wildtyp der Zelle.

In einem dritten wird das der Erfindung zu Grunde liegende Problem gelöst durch die Verwendung einer rekombinanten Zelle, bei der die Aktivität eines Polypeptides mit der SEQ ID NO 2 oder einer Variante davon gegenüber dem Wildtyp der Zelle verringert ist, zur Umsetzung eines Carbonsäureesters der Formel (I)

R¹ - A- COOR² (I),

wobei R¹ aus der Gruppe ausgewählt ist, die Wasserstoff, -CH₂OH, -CHO, -COOR³, -CH₂SH, - CH₂OR³ und -CH₂NH₂ umfasst,
wobei R² aus der Gruppe ausgewählt ist, die Wasserstoff und Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst,
und wobei es sich bei A um einen Alkylrest mit wenigstens acht Kohlenstoffatomen handelt.

In einer weiteren Ausführungsform des ersten, zweiten oder dritten Aspekts wird das Problem gelöst durch ein Verfahren oder eine Verwendung, wobei es sich bei A um einen gesättigten Alkylrest handelt, bevorzugt um einen Alkylrest der Formel -(CH₂)ₙ, wobei n wenigstens 4 ist. In einer weiteren Ausführungsform des ersten Aspekts wird das Problem gelöst durch ein Verfahren, wobei R¹ aus der Gruppe ausgewählt ist, die Wasserstoff, -CH₂OH, -CHO und -CH₂NH₂ umfasst.

In einer weiteren Ausführungsform des ersten Aspekts wird das Problem gelöst durch ein Verfahren, wobei es sich bei A um einen substituierten, nicht substituierten, linearen, verzweigten und/oder zyklischen Alkyl-, Aryl- oder Aralkylrest mit wenigstens 4, bevorzugter 6, noch bevorzugter 8 Kohlenstoffatomen handelt.

In einem vierten Aspekt wird das der Erfindung zu Grunde liegende Problem gelöst durch eine Zelle, die eine rekombinante Alkanhydroxylase exprimiert, wobei die Aktivität eines Polypeptides umfassend SEQ ID NO 2 oder eine Variante davon gegenüber dem Wildtyp der Zelle verringert ist.

In einer bevorzugten Ausführungsform des vierten Aspekts wird das Problem gelöst durch eine Zelle, wobei es sich bei der Alkanhydroxylase um eine Alkanhydroxylase aus der Gruppe umfassend AlkB-Monooxygenasen und Cytochrom P450-Monooxygenase der CYP153-Familie handelt.

In einem fünften Aspekt wird das der Erfindung zu Grunde liegende Problem gelöst durch eine Reaktionsmischung umfassend die Zelle gemäß dem vierten Aspekt oder einer seiner Ausführungsformen in wässriger Lösung sowie einen Carbonsäureester der Formel (I)

R¹ - A - COOR² (I),

wobei R¹ aus der Gruppe ausgewählt ist, die Wasserstoff, -CH₂OH, -CHO, -COOR³, -CH₂SH, - CH₂OR³ und -CH₂NH₂ umfasst,
wobei R² aus der Gruppe ausgewählt ist, die Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst, wobei es sich bei A um einen Alkylrest mit wenigstens vier Kohlenstoffatomen handelt,
wobei es sich bei A bevorzugt um einen gesättigten Alkylrest handelt, bevorzugt um einen Alkylrest der Formel -(CH₂)ₙ-, wobei n wenigstens 4 ist,
wobei R¹ bevorzugt aus der Gruppe ausgewählt ist, die Wasserstoff, -CH₂OH, -CHO und -CH₂NH₂ umfasst,
und wobei es sich bei A bevorzugt um einen substituierten, nicht substituierten, linearen, verzweigten und/oder zyklischen Alkyl-, Aryl- oder Aralkylrest mit wenigstens 4, bevorzugter 6, noch bevorzugter 8 Kohlenstoffatomen handelt.

In einer Ausführungsform des fünften Aspekts wird das Problem gelöst durch eine Reaktionsmischung, weiter umfassend eine hydrophoben organischen Lösung mit einem Kationenaustauscher.

In einer bevorzugten Ausführungsform des ersten bis fünften Aspekts wird das Problem gelöst durch ein Verfahren, eine Zelle oder eine Verwendung, wobei die Zelle weiterhin eine Transaminase exprimiert.

In einer bevorzugten Ausführungsform des ersten bis fünften Aspekts wird das Problem gelöst durch ein Verfahren, eine Zelle oder eine Verwendung, wobei die Zelle weiterhin eine Alanindehydrogenase exprimiert.

In einer bevorzugten Ausführungsform des ersten bis fünften Aspekts wird das Problem gelöst durch ein Verfahren, eine Zelle oder eine Verwendung, wobei die Zelle weiterhin ein Protein aus der AlkL-Familie aufweist.

In einer bevorzugten Ausführungsform des ersten bis fünften Aspekts wird das Problem gelöst durch ein Verfahren, eine Zelle oder eine Verwendung, wobei die Zelle eine ihrem Wildtyp gegenüber verringerte Aktivität wenigstens eines Enzyms aufweist, das eine der Reaktionen der β-Oxidation von Fettsäuren katalysiert, wobei es sich bevorzugt um ein Enzym aus der Gruppe handelt, die Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase, einen Fettsäureimporter oder Varianten davon umfasst, besonders bevorzugt um FadL oder eine Variante davon.

In einer bevorzugten Ausführungsform des ersten bis fünften Aspekts wird das Problem gelöst durch ein Verfahren, eine Zelle oder eine Verwendung, wobei die Zelle wenigstens ein Enzym aus der Gruppe umfassend Alkanhydroxylase, Alkoholdehydrogenase, Transaminase, Alanindehydrogenase und Protein aus der AlkL-Familie in rekombinanter Form aufweist und/oder überexprimiert.

In einer bevorzugten Ausführungsform des ersten bis fünften Aspekts wird das Problem gelöst durch ein Verfahren, eine Zelle oder eine Verwendung, wobei die Aktivität eines Polypeptides umfassend SEQ ID NO 2 oder eine Variante davon gegenüber dem Wildtyp der Zelle durch Knock out eines für ein Polypeptid umfassend SEQ ID NO 2 oder eine Variante davon kodierenden Gens verringert ist.

Die Erfindung beruht auf der überraschenden Erkenntnis der Erfinder, dass eine rekombinante Zelle, bei der die Aktivität eines Polypeptides umfassend SEQ ID NO 2 besonders oder einer Variante davon gegenüber dem Wildtyp der Zelle verringert ist, dahingehend für die Umsetzung von Carbonsäureestern geeignet ist, dass die Ausbeute, Kohlenstoff- und oder Stickstoffbilanz und Reinheit der daraus hervorgehenden Produkte überraschend höher ist als bei einer Zelle, die mit Hinblick auf das Polypeptid umfassend SEQ ID NO 2 die gleiche Aktivität wie der Wildtyp der Zelle aufweist. Dies trifft beispielsweise auf Reaktionen zu, bei denen der Carbonsäureester unter Verwendung von wenigstens einer Alkanhydroxylase und optional weiteren Enzymen oxidiert wird.

Die Erfindung beruht auf der weiteren überraschenden Erkenntnis der Erfinder, dass eine rekombinante Zelle, bei der die Aktivität eines Polypeptides umfassend SEQ ID NO 2 oder einer Variante davon gegenüber dem Wildtyp der Zelle verringert ist, besonders dahingehend für die Umsetzung von Carbonsäureestern geeignet ist, dass zur Aufarbeitung des Reaktionsproduktes aus der Umsetzung des Carbonsäureesters verwendete organische Lösungsmittel und flüssige Kationenaustauscher besonders effizient und/oder häufig durch Recycling für eine erneute Verwendung wiedergewonnen werden können, und dass die Abtrennung der wässrigen Reaktionsmischung von einer hydrophoben Lösung umfassend organisches Lösungsmittel und/oder flüssigen Kationenaustauscher verbessert ist.

Die Erfindung betrifft ein Verfahren zur Umsetzung eines Carbonsäureesters mittels einer Zelle, wobei es sich bei der Umsetzung um eine jegliche chemische Reaktion handeln kann, die von dem interessierenden Carbonsäureester als Edukt Gebrauch macht, und bei der die Hydrolyse oder vorzeitige Hydrolyse des Carbonsäureesters oder einer daraus hervorgehenden Verbindung die Ausbeute, Kohlenstoff- und/oder Stickstoffbilanz und/oder Reinheit des daraus herzustellenden Produktes beeinträchtigen könnte. Besonders geeignet sind die erfindungsgemäße Zelle und das erfindungsgemäße Verfahren für Umsetzungen, bei denen eine andere chemische Funktion als die Carbonsäureestergruppe umgesetzt wird, beispielsweise eine endständige Alkylgruppe, und bei denen es gilt, die Carbonsäureestergruppe zu erhalten. Erfindungsgemäß können jedoch auch Reaktionen wie Umesterungen der Carbonsäureestergruppe erfindungsgemäß durchgeführt werden, bei denen es gilt, eine vorzeitige und unspezifische Reaktion der Carbonsäureestergruppe zu vermeiden. Ebenso ist die erfindungsgemäße Lehre für Reaktionen geeignet, bei denen eine die Carbonsäureestergruppe enthaltende Verbindung nicht selbst das umzusetzende Edukt ist, sondern lediglich ihre Anwesenheit und Stabilität über einen längeren Zeitraum erforderlich ist, beispielsweise für den Fall, dass es sich um einen Induktor oder Aktivator eines Enzyms mit einer für das Verfahren essentiellen Aktivität handelt.

Essentiell für die Ausführung der Erfindung ist, dass die erfindungsgemäße Zelle oder in einem erfindungsgemäßen Verfahren verwendete Zelle eine rekombinante Zelle ist, bei der die Aktivität eines Polypeptides umfassend SEQ ID NO 2 oder eine Variante davon gegenüber dem Wildtyp der Zelle verringert ist. Diese Sequenz kodiert für BioH, ein Enzym, das für seine Fähigkeit bekannt ist, als Teil der Biosynthese von Biotin mit dem weiteren Enzym BioC Alanin und/oder Acetat in Pimeloyol-CoA zu überführen (Barker, D. F., and Campbell, A. M. (1980) J. Bacteriol. 143, 789-800). BioH wird in Escherichia *coli* von einer Nukleotidsequenz umfassend SEQ ID NO 1 kodiert. In einer bevorzugten Ausführungsform ist die Aktivität des Polypeptides umfassend SEQ ID NO 2 durch Knockout, beispielsweise teilweise Deletion, oder andere Maßnahmen zur Verringerung der Expression einer Nukleotidsequenz umfassend SEQ ID NO 1 oder eine Variante davon verringert.

Mit der Entwicklung moderner genetischer, mikrobiologischer und molekularbiologischer Methoden stehen dem Fachmann zahlreiche Werkzeuge zur Verfügung, mit denen er die Aktivität von in lebenden Zellen vorhandenen Polypeptiden routinemäßig messen und beeinflussen kann. Zur Bestimmung der Aktivität eines Enzyms, die in Form einer Suspension, eines Pellets vorliegt oder in prozessierter Form einer Zellkultur entnommen sein kann, können enzymatische Standardtests verwendet und ausgewertet werden, wie es in Lehrbüchern, beispielsweise Cornish-Bowden, 1995, beschrieben ist. Ein Assay zur Bestimmung der Aktivität des Polypeptides umfassend SEQ ID NO 1 oder eine Variante davon ist in X. Xie et al. (2007) Metabolic Engineering 9; 379-386 beschrieben.

Auch routinemäßig anwendbare Verfahren zur Verringerung der Aktivität eines Enzyms in einer Zelle, beispielsweise durch ungerichtete Mutagenese von Zellen durch Exposition gegenüber radioaktiver Strahlung gefolgt von Anreicherung oder Screening der Mutanten, durch ortsgerichtete Einführung von Punktmutationen oder durch Unterbrechung des Leserasters oder Deletion eines Teils des Leserasters eines chromosomal in eine Zelle integrierten für ein aktives Enzym kodierendes Gen sind im Stand der Technik beschrieben, beispielsweise in Maniatis *et al* (1989) oder in Fuchs & Schlegl (2007), und für den Fachmann routinemäßig durchführbar. Auch eine Aktivitätsverringerung auf der Basis von RNA-Interferenz (Tuschl, 2001) oder unter Verwendung von spezifischen Inhibitoren ist möglich. In einer bevorzugten Ausführungsform bedeutet die Formulierung "wobei die Zelle eine ihrem Wildtyp gegenüber verringerte Aktivität" eines Polypeptids aufweist, wie hierin verwendet, dass die Aktivität des Polypeptids in der veränderten Zelle gegenüber der Aktivität des gleichen Enzyms in einer Wildtyp-Zelle verringert ist. In einer bevorzugten Ausführungsform beträgt die relative Verringerung in der Reihenfolge zunehmender Bevorzugung 5, 10, 20, 40, 50, 75, 90, 95, 99 oder mehr Prozent der Aktivität. In einer besonders bevorzugten Ausführungsform ist keine Aktivität des Enzyms gegenüber dem Hintergrund mehr nachweisbar.

Besonders bevorzugt ist die Verringerung der Aktivität des Polypeptides umfassend SEQ ID NO 2 oder eine Variante davon durch einen Knock out. In einer bevorzugten Ausführungsform wird unter dem Begriff "Knock out", wie hierin verwendet, eine jegliche Maßnahme verstanden, die die Aktivität des Polypeptides umfassend SEQ ID NO 2 oder eine Variante davon dauerhaft und irreversibel, insbesondere auch bei Nachkommen entsprechender Zellen, verringert, bevorzugt durch Unterbrechung des Leserasters der für SEQ ID NO 2 oder eine Variante davon kodierenden Sequenz, durch Deletion wenigstens eines Teils der für SEQ ID NO 2 kodierenden Sequenz oder einer Variante davon, die einen Verlust der Enzymaktivität der kodierten Polypeptids bedingt, aber das Leseraster nicht unterbricht, oder durch Mutation einer für die Expression essentiellen Nukleotidsequenz, beispielsweise eines Promotors, einer Ribosomenbindestelle oder dergleichen. Maßnahmen zum Herstellen von Zellen mit verringerten Aktivitäten spezifischer Polypeptide sind dem Fachmann zugängliche Routineverfahren und im Stand der Technik hinlänglich beschrieben, beispielsweise in Kamionka et. al. (2005) Appl Environ Microbiol. 2005 February; 71(2): 728-733, Geng et. al. (2009), Journal of Biomedicine and Biotechnology Volume 2009, Article ID 646380, doi:10.1155/2009/646380, and Murphy (2009) Methods Mol Biol. 2011;765:27-42. Geeignet sind auch kommerziell erhältliche Kits, beispielsweise das TargeTron™ Gene Knockout System von Sigma Aldrich.

Die Erfindung betrifft Umsetzung eines Carbonsäureesters der Formel (I)

R¹ - A - COOR² (I),

wobei R¹ aus der Gruppe ausgewählt ist, die Wasserstoff, -CH₂OH, -CHO, -COOR³, -CH₂SH, -CH₂OR³ und -CH₂NH₂ umfasst, wobei R² aus der Gruppe ausgewählt ist, die Alkyl, bevorzugt Methyl, Ethyl und Propyl, umfasst, wobei R³ aus der Gruppe ausgewählt ist, die Wasserstoff und Alkyl, bevorzugt Methyl, Ethyl und Propyl, umfasst, und wobei es sich bei A um einen Alkylrest mit wenigstens vier Kohlenstoffatomen handelt. Bei dem Alkylrest A kann es sich, unter der Voraussetzung, dass er wenigstens vier Kohlenstoffatome umfasst, um einen beliebigen, insbesondere linearen, verzweigten oder zyklischen Alkylrest handelt. Der Alkylrest kann weiterhin gesättigt oder ungesättigt sein. In einer besonders bevorzugten Ausführungsform handelt es sich bei A um eine Alkylkette der Formel -(CH₂)ₙ-, wobei n 4, 5, 6, 7, 8, 9, 10, 11, 12, 13, 14, 15, 16, 17, 18, 19, 20, 21, 22, 23, 24, 25, 26, 27, 28, 29 oder 30 sein kann. In einer bevorzugtesten Ausführungsform ist stellt A einen Alkylrest der Formel -(CH₂)ₙ-dar, wobei n 4 bis 24, bevorzugter 4 bis 22 und am bevorzugtesten 4 bis 10 ist, R₁ ist Wasserstoff, -CH₂OH, -CHO, oder -COOH, am bevorzugtesten Wasserstoff, und R² ist Methyl oder Ethyl.

Im Zusammenhang mit Edukten der erfindungsgemäß umgesetzten Carbonsäureester sowie sämtlichen anderen hierin beschriebenen Verbindungen gilt, dass eine mit strukturellen Merkmalen, beispielsweise einer chemischen Formel, bezeichnete Verbindung gleichermaßen protonierte als auch deprotonierte oder andere dissoziiert Verbindungen bezeichnet. Beispielsweise wird unter dem Begriff "Acetat" gleichermaßen die protonierte Form, d. h. Essigsäure, als auch die dissoziiert Form, d.h. CH₃-COO⁻ verstanden.

Bei der erfindungsgemäß einzusetzenden Zelle handelt es sich bevorzugt um einen Ganzzellkatalysator in Form einer stoffwechselaktiven Zelle, die eine zur Umsetzung des Carbonsäureesters erforderliche enzymatische Aktivität aufweist, besonders bevorzugt durch Expression eines rekombinanten Enzyms. In einer weiteren bevorzugten Ausführungsform handelt es sich bei der Zelle um ein Lysat, ein Extrakt oder eine andere Präparation der Zelle, das wenigstens eine enzymatische Aktivität aufweist.

Mit Hinblick auf die Wahl des Organismus unterliegt die erfindungsgemäß benutzbare Zelle keinen Einschränkungen, sofern sie kultivierbar, stabil und Verfahren zur Abschwächung von Enzymaktivitäten, beispielsweise Knock outs, zugänglich ist. So kann es sich gleichermaßen um eine prokaryontische oder eukaryontische Zelle handeln. Im Falle einer eukaryontischen Zelle sind unizelluläre Eukaryonten besonders bevorzugt, besonders Hefen wie *Saccharomyces cerevisiae, Candida tropicalis, Candida albicans* und *Pichia pastoris.* Im Falle von prokaryontischen Zellen kann es sich beispielsweise um ein Bakterium handeln, das aus der Gruppe ausgewählt ist, die *Magnetococcus, Mariprofundus, Acetobacter, Acetobacterium, Acidiphilium, Afipia, Ahrensia, Asticcacaulis, Aurantimonas, Azorhizobium, Azospirillum, Bacillus, Bartonella, tribocorum, Beijerinckia, Bradyrhizobium, Brevundimonas, subvibrioides, Brucella, Caulobacter, Chelativorans, Citreicella, Citromicrobium, Clostridium, Corynebacterium, Dinoroseobacter, Erythrobacter, Fulvimarina, Gluconacetobacter, Granulibacter, Hirschia, Hoeflea, Hyphomicrobium, Hyphomonas, Ketogulonicigenium, Labrenzia, Loktanella, Magnetospirillum, Maricaulis, Maritimibacter, Mesorhizobium, Methylobacterium, Methylocystis, Methylosinus, Nitrobacter, Novosphingobium, Oceanibulbus, Oceanicaulis, Oceanicola, Ochrobactrum, Octadecabacter, Oligotropha, Paracoccus, Parvibaculum, Parvularcula, Pelagibaca, Phaeobacter, Phenylobacterium, Polymorphum, Pseudovibrio, Rhodobacter, Rhodomicrobium, Rhodopseudomonas, Rhodospirillum, Roseibium, Roseobacter, Roseomonas, Roseovarius, Ruegeria, Sagittula, Silicibacter, Sphingobium, Sphingomonas, Sphingopyxis, Starkeya, Sulfitobacter, Thalassiobium, Xanthobacter, Zymomonas, Agrobacterium, Rhizobium, Sinorhizobium, Anaplasma, Ehrlichia, Neorickettsia, Orientia, Rickettsia, Wolbachia, Bordetella, Burkholderia, Cupriavidus, Taiwanensis, Lautropia, Limnobacter, Polynucleobacter, Ralstonia, Chromobacterium, Eikenella, corrodens, Basfia, Kingella, Laribacter, Lutiella, Neisseria, Simonsiella, Achromobacter, Acidovorax, Alicycliphilus, Aromatoleum, Azoarcus, Comamonas, Dechloromonas, Delftia, Gallionella, Herbaspirillum, Herminümonas, Hylemonella, Janthinobacterium, Leptothrix, Methylibium, Methylobacillus, Methylophilales, Methyloversatilis, Methylovorus, Nitrosomonas, Nitrosospira, Oxalobacter, Parasutterella, Polaromonas, Polaromonas, Pusillimonas, Rhodoferax, Rubrivivax, Sideroxydans, Sutterella, wadsworthensis, Tayloreita, Thauera, Thiobacillus, Thiomonas, Variovorax, Verminephrobacter, Anaeromyxobacter, Bdellovibrio, bacteriovorus, Bilophila, Desulfarculus, Desulfatibacillum, Desulfobacca, Desulfobacterium, Desulfobulbus, Desulfococcus, Desulfohalobium, Desulfitobacterium, Desulfomicrobium, Desulfonatronospira, Desulfotatea, Desulfovibrio, Desulfuromonas, Geobacter, Haliangium, Hippea, Lawsonia, Myxococcus, Pelobacter, Plesiocystis, Sorangium, Stigmatella, Syntrophobacter, Syntrophus, Arcobacter, Caminibacter, Campylobacter, Helicobacter, Nitratifractor, Nitratiruptor, Sulfuricurvum, Sulfurimonas, Sulfurospirillum, Sulfurovum, Wolinella, Buchnera, Blochmannia, Hamiltonella, Regiella, Riesia, Citrobacter, Cronobacter, Dickeya, Edwardsiella, Enterobacter, Erwinia, Escherichia, Klebsiella, Pantoea, Pectobacterium, Proteus, Providencia, Rahnella, Salmonella, Serratia, Shigella, Sodalis, Wigglesworthia, Glossina, Xenorhabdus, Yersinia, Acidithiobacillus, Acinetobacter, Aeromonas, Alcanivorax, Alkalilimnicola, Allochromatium, Alteromonadales, Alteromonas, Baumannia, Beggiatoa, Bermanella, Carsonella, Ruthia, Vesicomyosocius, Cardiobacterium, Chromohatobacter, Colwellia, Congregibacter, Coxiella, Dichelobacter, Endoriftia, Enhydrobacter, Ferrimonas, Francisella, Glaciecola, Hahella, Ha*/*omonas, Halorhodospira, Halothiobacillus, Idiomarina, Kangiella, Legionella, Marinobacter, Marinomonas, Methylobacter, Methylococcus, Methylomicrobium, Methylophaga, Moraxella, Moritella, Neptunübacter, Nitrococcus, Pseudoalteromonas, Psychrobacter, Psychromonas, Reinekea, Rickettsiella, Saccharophagus, Shewanella, Succinatimonas, Teredinibacter, Thioalkalimicrobium, Thioalkalivibrio, Thiomicrospira, Tolumonas, Vibrionales, Actinobacillus, Aggregatibacter, Gallibacterium, Haemophilus, Histophilus, Mannheimia, Pasteurella, Azotobacter, Cellvibrio, Pseudomonas, Alüvibrio, Grimontia, Photobacterium, Photobacterium, Vibrio, Pseudoxanthomonas, Stenotrophomonas, Xanthomonas, Xyleila, Borrelia, Brachyspira, Leptospira, Spirochaeta, Treponema, Hodgkinia, Puniceispirillum, Liberibacter, Pelagibacter, Odyssella, Accumulibacter,* insbesondere *B. subtilis, B. megaterium, C. glutamicum, E. coli, Pseudomonas sp., Pseudomonas fluorescens, Pseudomonas putida, Pseudomonas stutzeri" Acinetobactersp., Burkholderia* sp., *Burkholderia thailandensis,* Cyanobakterien, *Klebsiella* sp., *Klebsiella oxytoca, Salmonella sp., Rhizobium sp.* und *Rhizobium meliloti,* umfasst. In einer besonders bevorzugten Ausführungsform handelt es sich um ein Enterobakterium, am bevorzugtesten um *Escherichia coli.*

Das erfindungsgemäße Verfahren erfordert, dass die Zelle mit dem Carbonsäureester in einer wässrigen Lösung kontaktiert wird. In einer bevorzugten Ausführungsform wird unter dem Begriff "Kontaktieren", wie hierin verwendet, verstanden, dass die erfindungsgemäße Zelle in unmittelbaren Kontakt mit dem jeweiligen Agens, beispielsweise dem Carbonsäureester oder einem flüssigen Kationenaustauscher gelangt, insbesondere ohne dass physikalische Barrieren wie poröse Membranen oder dergleichen zwischengeschaltet sind. Das Kontaktieren geschieht im einfachsten Fall dadurch, dass das Agens, beispielsweise der Carbonsäureester oder der flüssige Kationenaustauscher, zu einer wässrigen Lösung gegeben wird, in der sich die Zelle befindet.

Als wässrige Lösung können sämtliche Lösungen auf Wasserbasis verwendet werden, die zur Erhaltung oder Kultivierung der Zelle und/oder ihrer für die Umsetzung des Carbonsäureesters erforderlichen Aktivität geeignet sind. Darunter fallen gleichermaßen Kulturmedien für Mikroorganismen, darunter Vollmedien wie LB-Medien, Minimalmedien wie M9-Medien sowie Selektivmedien, beispielsweise solche, die eine hohe Salzkonzentration enthalten und daher nur das Wachstum halophiler oder zumindest halotoleranter Organismen ermöglichen. Besonders bevorzugt ist dies ein Minimalmedium, das möglichst wenige, vom Produkt der Umsetzung des Carbonsäureesters leicht abtrennbare Bestandteile enthält, um die Aufarbeitung des Produkts zu erleichtern.

Sofern das Produkt der vorgesehenen Umsetzung ausreichende Hydrophobizität und eine geeignete Ladung aufweist, bietet es sich an, es in einem Schritt b) durch Kontaktieren der wässrigen Lösung mit einer hydrophoben organischen Lösung umfassend einen Kationenaustauscher zu extrahieren. Geeignete Vorgehensweisen sind in der internationalen Patentanmeldung PCT/EP2011/071491 oder in der europäischen Patentanmeldung EP12181153.3 beschrieben. Kurz gesagt kann die wässrige Lösung während oder nach der Umsetzung mit einer hydrophoben Lösung umfassend einen Fettsäureester als Lösungsmittel und eine Fettsäure, bevorzugt eine ungesättigte Fettsäure, kontaktiert werden.

Bei der Einstellung der Temperatur und der Bedingungen bei Schritt a) sind die Ansprüche der Zelle, der Umsetzungsreaktion und benötigter Enzyme zu beachten. Die Temperaturansprüche verschiedener biotechnologisch bedeutsamer Zellen können mikrobiologischen und molekularbiologischen Lehrbüchern entnommen werden, z. B. Fuchs/Schlegl, Allgemeine Mikrobiologie, 2008, oder durch Wachstumsversuche im Rahmen von Routinearbeiten ermittelt werden. In einer bevorzugten Ausführungsform liegt der pH-Wert des wässrigen Kulturmediums zum Zeitpunkt des Kontaktierens zwischen 4 bis 9, bevorzugter zwischen 4,5 bis 8,5, am bevorzugtesten zwischen 6,5 und 7,5. In einer weiteren bevorzugten Ausführungsform liegt die Temperatur zwischen 0 und 45 °C, bevorzugter zwischen 15 und 40 °C, am bevorzugtesten zwischen 20 und 37 °C.

Bevorzugt wird für das erfindungsgemäße Verfahren eine Zelle verwendet, die eine rekombinante Alkanhydroxylase exprimiert, wobei die Aktivität eines Polypeptides umfassend SEQ ID NO 2 oder eine Variante davon gegenüber dem Wildtyp der Zelle verringert ist. In einer bevorzugten Ausführungsform handelt es sich bei der Alkanhydroxylase um eine Cytochrom P450-Monooxygenase der CYP153-Familie. In einer bevorzugten Ausführungsform wird unter dem Begriff "Cytochrom P450-Monooxygenase der CYP153-Familie" eine cytosolische Oxidase verstanden, die Teil eines 3 Komponenten-Systems ist, das weiterhin ein Ferredoxin und eine Ferredoxin-Reduktase umfasst, mit einer Alkan-Bindestelle und der Fähigkeit, Alkane zu hydroxylieren. In einer besonders bevorzugten Ausführungsform handelt es sich um ein Enzym, das zu wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist oder um ein Enzym, das eine Polypeptidsequenz umfasst, die wenigstens 80, bevorzugt 90, am bevorzugtesten 95 oder 99 Prozent Sequenzidentität zur Cytochrom P450-Monooxygenase der CYP153-Familie aus *Alcanivorax borkumensis* SK2 (Datenbankcode YP_691921) aufweist und darüber hinaus Alkanhydroxylase-Aktivität aufweist. Die genannten Datenbankcodes beziehen sich dabei, wie durchgehend in dieser Anmeldung, auf die NCB (National Center for Biotechnology Information, Bethesda, USA)I-Datenbanken, genauer gesagt die am 8. November 2012 online verfügbare Version. In einer bevorzugten Ausführungsform ist unter dem Begriff "Alkanhydroxylase-Aktivität", wie hierin verwendet, die Fähigkeit zu verstehen, die Hydroxylierung von Alkanen oder unsubstituierten linearen Alkylresten umfassend wenigstens sechs, bevorzugt zwölf Kohlenstoffstoffreste zu katalysieren. In einer weiteren bevorzugten Ausführungsform wird unter dem Begriff "Cytochrom P450-Monooxygenase der CYP153-Familie" eine nicht membrangebundene Oxidase verstanden, die eine Bindestelle für Alkane, unsubstituierte lineare Alkylreste umfassend wenigstens fünf, bevorzugt zwölf Kohlenstoffstoffreste oder einfach hydroxylierte Alkane und deren Polypeptidkette das Motiv LL(I/L)(V/I)GGNDTTRN umfasst. In einer bevorzugten Ausführungsform handelt es sich bei einer "Cytochrom P450-Monooxygenase der CYP153-Familie", wie hierin verwendet, um eine Cytochrom P450-Monooxygenase der CYP153-Familie aus *Alcanivorax borkumensis* SK2 (Datenbankcode YP_691921) oder eine Variante, die bevorzugt Alkanhydroxylaseaktivität aufweist.

Bei den erfindungsgemäß verwendeten Enzymen handelt es sich bevorzugt um rekombinante Enzyme. In einer bevorzugten Ausführungsform wird unter dem Begriff "rekombinant", wie hierin verwendet, verstanden, dass das entsprechende Nukleinsäure-Molekül in der natürlichen Zelle nicht vorkommt und/oder es unter Verwendung von gentechnischen Methoden hergestellt wurde. In einer bevorzugten Ausführungsform spricht man von einem rekombinanten Protein, wenn das entsprechende Polypeptid von einer rekombinanten Nukleinsäure kodiert ist. In einer bevorzugten Ausführungsform wird unter einer rekombinanten Zelle, wie hierin verwendet, eine Zelle verstanden, die wenigstens eine rekombinante Nukleinsäure oder ein rekombinantes Polypeptid aufweist. Dem Fachmann sind zum Herstellen rekombinanter Moleküle oder Zellen geeignete Verfahren bekannt, beispielsweise die in Sambrook/Fritsch/Maniatis (1989): Molecular Cloning: A Laboratory Manual, Cold Spring Harbor Laboratory Press, 2nd edition, beschriebenen. Rekombinante Enzyme werden bevorzugt überexprimiert, beispielsweise unter Verwendung von pET- oder pGEX-Vektor-Systemen, die dem Fachmann bekannt sind.

Zur optimalen Versorgung der Cytochrom P450-Monooxygenase der CYP153-Familie mit Elektronen aus dem Reduktionsmittel, bevorzugt NADH, ist es bevorzugt, dass die Zelle die Monoxygenase zusammen mit funktionell mit ihr wechselwirkender Ferredoxin-Reduktase und funktionell mit ihr wechselwirkendem Ferredoxin exprimiert wird. Dabei kann es sich um isolierte oder bei der Verwendung eines Ganzzellkatalysators um co-exprimierte Polypeptide oder um N-oder C-terminal mit der Cytochrom P450-Monooxygenase der CYP153-Familie fusionierte Polypeptide handeln. Ob eine Ferredoxin-Reduktase oder ein Ferredoxin mit einer gegebenen Cytochrom P450-Monooxygenase der CYP153-Familie mit einander funktionell wechselwirken, kann der Fachmann leicht dadurch feststellen, ob das Reduktionsmittel in Gegenwart eines Alkansubstrates und der drei Polypeptide oxidiert wird. Alternativ kann der von Scheps, D., Malca, H., Hoffmann, B., Nestl, B. M, und Hauer, B. (2011) Org. Biomol. Chem., 9, 6727 beschriebene Enzymtest verwendet werden, der im Fall funktionell wechselwirkender Polypeptide eine deutliche Erhöhung der Reaktionsgeschwindigkeit zeigt. In einer besonders bevorzugten Ausführungsform stammen die Cytochrom P450-Monooxygenase der CYP153-Familie, das Ferredoxin und die Ferredoxin-Reduktase aus dem gleichen Organismus. In einer besonders bevorzugten Ausführungsform handelt es sich um die Ferredoxin-Reduktase aus *Alcanivorax borkumensis* SK2 (Datenbankcode YP_691923) oder eine Variante davon, das Ferredoxin aus *Alcanivorax borkumensis* SK2 (Datenbankcode YP_691920) oder eine Variante davon und die Cytochrom P450-Monooxygenase der CYP153-Familie aus *Alcanivorax borkumensis* SK2 (Datenbankcode YP_691921) oder eine Variante davon.

In einer weiteren bevorzugten Ausführungsform handelt es sich bei der Alkanhydroxylase um eine AlkB-Monooxygenase. AlkB stellt eine zunächst aus dem AlkBGT-System aus *Pseudomonas putida* Gpo1 bekannt gewordene Oxidoreduktase dar, die von zwei weiteren Polypeptiden, AlkG und AlkT, abhängig ist. AlkT wird als FAD-abhängige Rubredoxin-Reduktase charakterisiert, die Elektronen aus NADH an AlkG weitergibt. Bei AlkG handelt es sich um ein Rubredoxin, ein eisenhaltiges Redoxprotein, das als direkter Elektronendonor für AlkB fungiert. In einer bevorzugten Ausführungsform wird unter dem Begriff "AlkB-Monooxygenase" ein Polypeptid mit einer Sequenzhomologie von wenigstens, in der Reihenfolge zunehmender Bevorzugung angegeben, 75, 80, 85, 90, 92, 94, 96, 98 oder 99 % zur Sequenz des AlkB von *Pseudomonas putida* Gpo1 (Datenbankcode: CAB54050.1; dieser Datenbankcode stammt wie alle weiteren in der Anmeldung verwendeten aus dem Stand der Technik, nämlich aus der NCBI Datenbank, genauer dem am 15. Oktober 2012 online verfügbaren Release) mit der Fähigkeit, Alkane zu oxidieren. In einer besonders bevorzugten Ausführungsform handelt es sich bei der AlkB-Monooxygenase um eine mit den AlkG (CAB54052.1)- und AlkT (CAB54063.1)-Polypeptiden aus *Pseudomonas putida* Gpo1 funktionell zusammenwirkende, Alkane oxidierende Oxidoreduktase. Zur optimalen Versorgung der AlkB-Alkanhydroxylase mit Elektronen ist es bevorzugt, dass die Zelle die Monoxygenase zusammen mit funktionell mit ihr wechselwirkenden Hilfsproteinen, bevorzugt AlkG und/oder AlkT oder jeweils Varianten davon exprimiert wird, wobei es sich in einer besonders bevorzugten Ausführungsform wiederum um AlkG (CAB54052.1)- und AlkT (CAB54063.1)-Polypeptide aus *Pseudomonas putida* Gpo1 handelt.

Die Fähigkeit der erfindungsgemäßen oder im erfindungsgemäßen Verfahren verwendeten Zelle zur Oxidation von Substraten kann dadurch verstärkt werden, dass die Zelle alternativ oder zusätzlich zur Alkanhydroxylase eine Alkoholdehydrogenase exprimiert. In einer bevorzugten Ausführungsform wird unter dem Begriff "Alkoholdehydrogenase", wie hierin verwendet, wird ein Enzym verstanden, das einen Aldehyd bzw. Keton zu dem entsprechenden primären bzw. sekundären Alkohol oxidiert. Beispiele umfassend die Alkoholdehydrogenasen von *Ralstonia eutropha* (ACB78191.1), *Lactobacillus brevis* (YP_795183.1), *Lactobacillus kefiri* (ACF95832.1), aus Pferdeleber, von *Paracoccus pantotrophus* (ACB78182.1) und *Sphingobium yanoikuyae* (EU427523.1) sowie die jeweiligen Varianten davon.

Bei der Verwendung eines Ganzzellkatalysators kann sich das Problem stellen, dass ein Substrat mit einem intrazellulär lokalisierten Enzym in Kontakt gebracht werden muss, damit es zur erwünschten Reaktion kommt. Im Falle langkettiger Alkane und Derivate davon ist bevorzugt, dass der Ganzzellkatalysator ein Polypeptid der AlkL-Familie aufweist. In einer bevorzugten Ausführungsform handelt es sich bei einem "Polypeptid der AlkL-Familie", wie hierin verwendet, um ein Polypeptid, das über eine Länge von 230 aufeinander abfolgenden Aminosäuren eine wenigstens 80, bevorzugt 90, noch bevorzugter 90%ige Sequenzidentität zu AlkL aus *Pseudomonas putida* (Datenbankcode CAB69081) oder eine Variante von AlkL aus *Pseudomonas putida* und bevorzugt die Fähigkeit aufweist, den Import von langkettigen Alkanen ins Innere einer Zelle zu unterstützen. In einer weiteren Ausführungsform handelt es sich bei einem "Polypeptid der AlkL-Familie", wie hierin verwendet, um eine in der äußeren Membran eines Gram-negativen Bakteriums lokalisiertes Polypeptid, welches das Sequenzmotiv DXWAPAXQ(V/A)GXR, aufweist, wobei X eine proteinogene Aminosäure darstellt, und bevorzugt zusätzlich AlkL aus *Pseudomonas putida* (Datenbankcode CAB69081) oder eine Variante davon ist. Beispielhafte Mitglieder der AlkL-Familie umfassen AlkL aus *Pseudomonas putida* (Datenbankcode CAB69081), *Marinobacter aquaeolei* VT8 (Datenbankcode YP_957722), *Oceanicaulis alexandrii* HTCC2633 (Datenbankcode ZP_00953584), *Marinobactermanganoxydans* MnI7-9 (Datenbankcode ZP_09158756), *Caulobacter* sp*.* K31 (Datenbankcode YP_001672217), *Pseudomonas oleovorans* (Datenbankcode Q00595) und Varianten davon.

Die Lehre der vorliegenden Erfindung kann nicht nur unter Verwendung von Makromolekülen mit der exakten Aminosäure- oder Nukleinsäuresequenz, auf die hierin Bezug genommen wird, bzw. nicht nur unter Verwendung von einer Zelle mit relativ zum jeweiligen Wildtyp verringerter Aktivität eines Polypeptides mit der exakten Aminosäuresequenz, auf die hierin Bezug genommen wird, ausgeführt werden, sondern auch unter Verwendung einer Variante derartiger Makromoleküle oder von einer Zelle mit einer relativ zum jeweiligen Wildtyp der jeweiligen Zelle verringerten Aktivität einer Variante des Polypeptids, die durch Deletion, Addition oder Substitution einer oder mehr als einer Aminosäuren oder Nukleinsäuren erhalten werden kann. In einer bevorzugten Ausführungsform bedeutet der Begriff "Variante" einer Nukleinsäuresequenz oder Aminosäuresequenz, im Folgenden gleichbedeutend und austauschbar mit dem Begriff "Homologon" gebraucht, wie hierin verwendet, eine andere Nukleinsäure- oder Aminosäuresequenz, die mit Hinblick auf die entsprechende ursprüngliche Wildtyp-Nukleinsäure- oder -aminosäuresequenz eine Homologie, hier gleichbedeutend mit Identität verwendet, von 70, 75, 80, 85, 90, 92, 94, 96, 98, 99 % oder mehr Prozent aufweist, wobei bevorzugt andere als die das katalytisch aktive Zentrum ausbildende Aminosäuren oder für die Struktur oder Faltung essentielle Aminosäuren deletiert oder substituiert sind oder solche lediglich konservativ substituiert sind, beispielsweise ein Glutamat statt einem Aspartat oder ein Leucin statt einem Valin. Der Stand der Technik beschreibt Algorithmen, die verwendet werden können, um das Ausmaß von Homologie von zwei Sequenzen zu berechnen, z. B. Arthur Lesk (2008), Introduction to bioinformatics, 3^{rd} edition. In einer weiteren bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche enzymatische Aktivität des Wildtypmoleküls bzw. des ursprünglichen Moleküls auf. Zum Beispiel weist eine Variante eines als Protease enzymatisch aktiven Polypeptids die gleiche oder im Wesentlichen die gleiche proteolytische Aktivität wie das Polypeptidenzym auf, d.h. die Fähigkeit, die Hydrolyse einer Peptidbindung zu katalysieren. In einer besonderen Ausführungsform bedeutet der Begriff "im Wesentlichen die gleiche enzymatische Aktivität" eine Aktivität mit Hinblick auf die Substrate des Wildtyp-Polypeptids, die deutlich über der Hintergrundaktivität liegt oder/und sich um weniger als 3, bevorzugter 2, noch bevorzugter eine Größenordnung von den K_{M}- und/oder k_{cat}- Werten unterscheidet, die das Wildtyppolypeptid mit Hinblick auf die gleichen Substrate aufweist. In einer weiteren bevorzugten Ausführungsform umfasst der Begriff "Variante" einer Nukleinsäure- oder Aminosäuresequenz wenigstens einen aktiven Teil/oder Fragment der Nukleinsäure- bzw. Aminosäuresequenz. In einer weiteren bevorzugten Ausführungsform bedeutet der Begriff "aktiver Teil", wie hierin verwendet, eine Aminosäuresequenz oder eine Nukleinsäuresequenz, die eine geringere als die volle Länge der Aminosäuresequenz aufweist bzw. für eine geringere als die volle Länge der Aminosäuresequenz kodiert, wobei die Aminosäuresequenz oder die kodierte Aminosäuresequenz mit geringerer Länge als der Wildtyp-Aminosäuresequenz im Wesentlichen die gleiche enzymatische Aktivität wie das Wildtyppolypeptid oder eine Variante davon aufweist, beispielsweise als Protease. In einer besonderen Ausführungsform umfasst der Begriff "Variante" einer Nukleinsäure eine Nukleinsäure, deren komplementärer Strang, bevorzugt unter stringenten Bedingungen, an die Wildtyp-Nukleinsäure bindet. Die Stringenz der Hybridisierungsreaktion ist für den Fachmann leicht bestimmbar und hängt im Allgemeinen von der Länge der Sonde, den Temperaturen beim Waschen und der Salzkonzentration ab. Im Allgemeinen benötigen längere Sonden höhere Temperaturen zum Hybridisieren, wohingegen kürzere Proben mit geringen Temperaturen auskommen. Ob Hybridisierung stattfindet, hängt im Allgemeinen von der Fähigkeit der denaturierten DNA ab, an komplementäre Stränge zu annellieren, die in ihrer Umgebung vorhanden sind, und zwar unterhalb der Schmelztemperatur. Die Stringenz von Hybridisierungsreaktion und entsprechende Bedingungen sind ausführlicher in F M Ausubel (1995), Current Protocols in Molecular Biology. John Wiley & Sons, Inc. beschrieben. Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet in einer bevorzugten Ausführungsform unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996). Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von in der Reihenfolge zunehmender Bevorzugung ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2 x SSC oder 0,1 x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise in der Reihenfolge zunehmender Bevorzugung mindestens 70% oder mindestens 80% oder mindestens 90%, mindestens 91 %, mindestens 92%, mindestens 93%, mindestens 94%, mindestens 95%, mindestens 96%, mindestens 97%, mindestens 98% oder mindestens 99% Identität zur Sequenz des eingesetzten Nukleinsäuremoleküls. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558). In einer bevorzugten Ausführungsform umfasst der Begriff "Variante" einer Nukleinsäure, wie hierin verwendet, eine beliebige Nukleinsäuresequenz, die für die gleiche Aminosäuresequenz wie die ursprüngliche Nukleinsäure oder eine Variante dieser Aminosäuresequenz im Rahmen der Degeneriertheit des genetischen Codes kodiert.

In einer bevorzugten Ausführungsform weist die erfindungsgemäß verwendete Zelle eine ihrem Wildtyp gegenüber verringerte Aktivität wenigstens eines Enzyms aufweist, das eine der Reaktionen der β-Oxidation von Fettsäuren katalysiert, wobei es sich bevorzugt um ein Enzym aus der Gruppe handelt, die Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase, einen Fettsäureimporter oder Varianten davon umfasst, besonders bevorzugt um FadL oder eine Variante davon. Die β-Oxidation von Fettsäuren ist ein weit verbreiteter Stoffwechselweg, der es prokaryontischen und eukaryontischen Organismen gleichermaßen erlaubt, Fettsäuren zu oxidieren und die darin enthaltene chemische Energie dem Stoffwechsel verfügbar zu machen. Im weiteren Sinn beginnt sie mit der Aufnahme einer Fettsäure in die Zelle, im Falle von *E. coli* durch den Transporter FadL, der sie durch die äußere bzw. innere Membran der Gram-negativen Bakterienzelle schleust und das FadD -Genprodukt, das die Fettsäure in Form des CoA-Esters ins Cytosol freisetzt. Dort wird die Fettsäure, sofern die Bedingungen es erfordern, zunächst an der β-Position des CoA-Fettsäureesters durch eine Acyl-CoA-Dehydrogenase, im Falle von *E. coli* FadE, oxidiert. Ein ähnliches Molekül kann alternativ auch aus einer doppelt ungesättigten Fettsäure durch Reduktion mittels einer 2,4-dienoyl-CoA-Reduktase, bei *E. coli* FadH, gebildet werden. Ein multifunktionelles Enzym, die Enoyl-CoA-Hydratase/3-Hydroxyacyl-CoA-Dehydrogenase, bei *E. coli* FadB, katalysiert anschließend die Hydratisierung unter Bildung des sekundären Alkohols und dessen anschließende Oxidation zum Keton. Im letzten Schritt katalysiert eine 3-Ketoacyl-CoA-Thiolase, im Falle von *E. coli* FadA, die Spaltung des Ketoacyl-CoA mit dem Ergebnis, dass Acetyl-CoA und ein im Vergleich zum Ausgangsmolekül um zwei Kohlenstoffatome verkürzter CoA-Ester der Fettsäure freigesetzt werden. Sofern es sich nicht ebenfalls um Acetyl-CoA handelt, kann letzterer erneut in den β-Oxidationszyklus eingespeist und unter Oxidation verkürzt werden. An der Regulation der β-Oxidation von Fettsäuren ist auch FadR beteiligt, ein Regulator des Fad-Operons, der die für den Abbau von Fettsäuren erforderlichen Gene umfasst, ohne dass FadR eine Reaktion der β-Oxidation katalysieren würde. In einer bevorzugten Ausführungsform wird unter dem Begriff "Enzym, das eine der Reaktionen der β-Oxidation von Fettsäuren katalysiert" ein jegliches Enzym verstanden, das direkt mit dem Fettsäuresubstrat oder einem auf dem Weg zum Acetyl-CoA daraus entstehenden Molekül wechselwirkt, bevorzugt es als Substrat erkennt, und seine Umwandlung zu einem auf diesem Abbauweg näher am Acetyl-CoA liegenden Stoffwechselprodukt katalysiert, bevorzugt einschließlich des Fettsäureimporters, der die Aufnahme der Fettsäure in die Zelle bewerkstelligt. Beispielsweise zählt zu diesen Enzymen nach der vorangegangenen Definition die Acyl-CoA-Dehydrogenase, da sie mit dem Fettsäure-CoA-Ester wechselwirkt und dessen Umwandlung zum Enyol-CoA katalysiert, das auf dem Stoffwechselweg der β-Oxidation näher am Acetyl-CoA liegt als der Fettsäure-CoA-Ester. In einer besonders bevorzugten Ausführungsform wird unter dem Begriff "Enzym, das eine der Reaktionen der β-Oxidation von Fettsäuren katalysiert", wie hierin verwendet, jedes Enzym aus der Gruppe verstanden, die die Genprodukte FadA, FadB, FadD, FadL und FadE aus E. *coli* und/oder deren Varianten oder Homologa aus anderen Organismen umfasst. Die Genprodukte FadA, FadB, FadD, FadL und FadE aus *E. coli* ebenso wie Varianten und Homologa aus zahlreichen weiteren biotechnologisch nutzbaren Organismen und ihre Nukleinsäure- und Polypeptidssequenzen sind im Stand der Technik beschrieben, beispielsweise FadA unter Zugangsnummer AP009048.1, FadB unter Zugangsnummer BAE77457.1, FadD unter Zugangsnummer BAA15609.1, FadE unter Zugangsnummer BAA77891.2 und FadL unter Zugangsnummer BAA16205.1.

In einer weiteren bevorzugten Ausführungsform exprimiert die erfindungsgemäße oder im erfindungsgemäßen Verfahren verwendete Zelle eine Transaminase. In einer bevorzugten Ausführungsform wird unter dem Begriff "Transaminase", wie hierin verwendet, ein Enzym verstanden, das die Übertragung von α-Aminogruppen von einem Donor-, bevorzugt einer Aminosäure, auf ein Akzeptormolekül, bevorzugt eine α-Ketocarbonsäure, katalysiert. Beispielsweise kann eine Transaminase aus der Gruppe umfassend 3HMU_A, AAD41041.1, AAK15486.1, ABE03917.1, ADR60699.1, ADR61066.1, ADR62525.1, AEL07495.1, CAZ86955.1, EFW82310.1, EFW87681.1, EGC99983.1, EGD03176.1, EGE58369.1, EGH06681.1, EGH08331.1, EGH24301.1, EGH32343.1, EGH46412.1, EGH55033.1, EGH62152.1, EGH67339.1, EGH70821.1, EGH71404.1, EGH78772.1, EGH85312.1, EGH97105.1, EGP57596.1, NP_102850.1, NP_106560.1, NP_248912.1, NP_248990.1, NP_354026.2, NP_421926.1, NP_637699.1, NP_642792.1, NP_744329.1, NP_744732.1, NP_747283.1. NP_795039.1, NP_901695.1 (, XP_002943905.1, YP_001021095.1, YP_001059677.1, YP_001061726.1, YP_001066961.1, YP_001074671.1, YP_001120907.1, YP_001140117.1, YP_001170616.1, YP_001185848.1, YP_001188121.1, YP_001233688.1, YP_001268866.1, YP_001270391.1, YP_001345703.1, YP_001412573.1, YP_001417624.1, YP_001526058.1, YP_001579295.1, YP_001581170.1, YP_001668026.1, YP_001669478.1, YP_001671460.1, YP_001685569.1, YP_001747156.1, YP_001749732.1, YP_001765463.1, YP_001766294.1, YP_001790770.1, YP_001808775.1, YP_001809596.1, YP_001859758.1, YP_001888405.1, YP_001903233.1, YP_001977571.1, YP_002229759.1, YP_002231363.1, YP_002280472.1, YP_002297678.1, YP_002543874.1, YP_002549011.1, YP_002796201.1, YP_002801960.1, YP_002875335.1, YP_002897523.1, YP_002912290.1, YP_002974935.1, YP_003060891.1, YP_003264235.1, YP_003552364.1, YP_003578319.1, YP_003591946.1, YP_003607814.1, YP_003641922.1, YP_003674025.1, YP_003692877.1, YP_003755112.1, YP_003896973.1, YP_003907026.1, YP_003912421.1, YP_004086766.1, YP_004142571.1, YP_004147141.1, YP_004228105.1, YP_004278247.1, YP_004305252.1, YP_004356916.1, YP_004361407.1, YP_004378186.1, YP_004379856.1, YP_004390782.1, YP_004472442.1, YP_004590892.1, YP_004612414.1, YP_004676537.1, YP_004693233.1, YP_004701580.1, YP_004701637.1, YP_004704442.1, YP_108931.1, YP_110490.1, YP_168667.1, YP_237931.1, YP_260624.1, YP_262985.1, YP_271307.1, YP_276987.1, YP_334171.1, YP_337172.1, YP_350660.1, YP_351134.1, YP_364386.1, YP_366340.1, YP_369710.1, YP_370582.1, YP_426342.1, YP_440141.1, YP_442361.1, YP_468848.1, YP_521636.1, YP_554363.1, YP_608454.1, YP_610700.1, YP_614980.1, YP_622254.1, YP_625753.1, YP_680590.1, YP_751687.1, YP_767071.1, YP_774090.1, YP_774932.1, YP_788372.1, YP_858562.1, YP_928515.1, YP_983084.1, YP_995622.1, ZP_00948889.1, ZP_00954344.1, ZP_00959736.1, ZP_00998881.1, ZP_01011725.1, ZP_01037109.1, ZP_01058030.1, ZP_01076707.1, ZP_01103959.1, ZP_01167926.1, ZP_01224713.1, ZP_01442907.1, ZP_01446892.1, ZP_01550953.1, ZP_01625518.1, ZP_01745731.1, ZP_01750280.1, ZP_01754305.1, ZP_01763880.1, ZP_01769626.1, ZP_01865961.1, ZP_01881393.1, ZP_01901558.1, ZP_02145337.1, ZP_02151268.1, ZP_02152332.1, ZP_02167267.1, ZP_02190082.1, ZP_02242934.1, ZP_02360937.1, ZP_02367056.1, ZP_02385477.1, ZP_02456487.1, ZP_02883670.1, ZP_03263915.1, ZP_03263990.1, ZP_03400081.1, ZP_03452573.1, ZP_03456092.1, ZP_03517291.1, ZP_03529055.1, ZP_03571515.1, ZP_03572809.1, ZP_03587785.1, ZP_03588560.1, ZP_03697266.1, ZP_03697962.1, ZP_04521092.1, ZP_04590693.1, ZP_04890914.1, ZP_04891982.1, ZP_04893793.1, ZP_04902131.1, ZP_04905327.1, ZP_04941068.1, ZP_04944536.1, ZP_04945255.1, ZP_04959332.1, ZP_04964181.1, ZP_05053721.1, ZP_05063588.1, ZP_05073059.1, ZP_05077806.1, ZP_05082750.1, ZP_05091128.1, ZP_05095488.1, ZP_05101701.1, ZP_05116783.1, ZP_05121836.1, ZP_05127756.1, ZP_05637806.1, ZP_05742087.1, ZP_05783548.1, ZP_05786246.1, ZP_05843149.1, ZP_05945960.1, ZP_06459045.1, ZP_06487195.1, ZP_06492453.1, ZP_06493162.1, ZP_06703644.1, ZP_06731146.1, ZP_06839371.1, ZP_07007312.1, ZP_07266194.1, ZP_07374050.1, ZP_07662787.1, ZP_07778196.1, ZP_07797983.1, ZP_08099459.1, ZP_08138203.1, ZP_08141719.1, ZP_08142973.1, ZP_08177102.1, ZP_08185821.1, ZP_08186468.1, ZP_08208888.1, ZP_08266590.1, ZP_08402041.1, ZP_08406891.1, ZP_08522175.1, ZP_08527488.1, ZP_08631252.1, ZP_08636687.) verwendet werden.

In einer weiteren bevorzugten Ausführungsform exprimiert die erfindungsgemäße oder im erfindungsgemäßen Verfahren verwendete Zelle eine Alanindehydrogenase. In einer bevorzugten Ausführungsform wird unter dem Begriff "Alanindehydrogenase", wie hierein verwendet, ein Enzym verstanden, das die Umwandlung von L-Alanin unter Verbrauch von Wasser und NAD⁺ zu Pyruvat, Ammoniak und NADH katalysiert. Beispielsweise können die Alanindehydrogenasen aus der Gruppe umfassend die Alanindehydrogenase aus *Bacillus subtilis* (Datenbankcode L20916), *Rhizobium leguminosarum* (Datenbankcode CP001622), *Vibrio proteolyticus* (Datenbankcode AF070716), *Mycobacterium tuberculosis* (Datenbankcode X63069), *Enterobacter aerogenes* (Datenbankcode AB013821), EGR93259.1, YP_003654745.1, YP_003651439.1, YP_003637111.1, YP_003631815.1, YP_001327051.1, YP_001262560.1, YP_886996.1, YP_882850.1, YP_704410.1, YP_703508.1, ZP_08624689.1, YP_001230376.1, P17557.1, P17556.1, CCB94892.1, CCB73698.1, YP_001168635.1, YP_004668736.1, YP_004569425.1, YP_003513168.1, YP_004561169.1, ZP_08554945.1, YP_400777.1, ZP_08311476.1, ZP_08310170.1, ZP_08267322.1, ZP_08263846.1, ZP_07898723.1, YP_149301.1, YP_148605.1, YP_004340432.1, EFT09946.1, EFS80513.1, EFS51332.1, EFS42459.1, YP_003060895.1, YP_003059033.1, ZP_03305373.1, YP_847214.1, YP_004095847.1, YP_003338282.1, YP_003337256.1, YP_355846.1, YP_253131.1, ZP_08197563.1, ZP_08196283.1, ADW06447.1, YP_734091.1, NP_372233.1, NP_102173.1, ZP_08170259.1, EGD36706.1, EGD32748.1, ZP_08155540.1, YP_004142849.1, YP_002417649.1, YP_001301040.1, YP_002992892.1, YP_081348.1, YP_080482.1, YP_002476349.1, ZP_08115025.1, ZP_08114403.1, YP_003552869.1, YP_002358112.1, YP_575010.1, YP_477594.1, YP_474564.1, YP_130399.1, YP_129373.1, YP_123314.1, NP_810467.1, NP_646469.1, NP_626044.1, NP_391071.1 (kodiert durch SEQ ID NR: 11), ZP_08086822.1, ZP_08084776.1, ZP_08083119.1, ZP_08020768.1, ZP_08013590.1, ZP_08011832.1, YP_003783744.1, YP_002781576.1, YP_002780533.1, ZP_02195873.1, NP_797482.1, ZP_07645051.1, ZP_07643260.1, ZP_06611917.1, AAT40119.1, ZP_07864946.1, YP_004068409.1, YP_002796203.1, YP_002774420.1, YP_003600348.1, YP_003599946.1, YP_003565624.1, YP_003565223.1, YP_335198.1, YP_423850.1, YP_155059.1, ZP_07843538.1, ZP_07841226.1, ZP_06928932.1, ZP_05692073.1, ZP_05687006.1, ZP_04867480.1, YP_775531.1, CBE70214.1, ZP_07721182.1, ZP_04302850.1, ZP_04298961.1, ZP_04287684.1, ZP_04277177.1, ZP_04248389.1, ZP_04235899.1, ZP_02159718.1, ZP_02152178.1, YP_003974610.1, YP_003546595.1, YP_002317127.1, ZP_07313778.1, ZP_07302778.1, ZP_07298850.1, CBK69442.1, YP_003413835.1, YP_003595089.1, ZP_06807811.1, YP_003582455.1, YP_003464731.1, YP_003496397.1, YP_003421918.1, CBL07274.1, CBK64956.1, YP_003508515.1, AAL87460.1, AAC23579.1, AAC23578.1, AAC23577.1, ACU78652.1, YP_003471439.1, YP_003452777.1, ZP_06384971.1, ACY25368.1, ABC26869.1, AAP44334.1, EEZ80018.1, ZP_05110458.1, 1PJB_A, ZP_04717201.1, ZP_04689103.1, CAO90307.1, CAM75354.1, CAA44791.1, BAA77513.1, EGR96638.1, EGL90046.1, YP_004510847.1, ZP_08450330.1, YP_003387804.1, YP_003058152.1, EFS74272.1, EFS67128.1, ZP_06844564.1, YP_826658.1, YP_001195249.1, YP_003095978.1, YP_469292.1, YP_004442054.1, YP_004461174.1, YP_004055616.1, YP_003576656.1, YP_003094537.1, YP_001295973.1, AEE71143.1, YP_004447480.1, YP_003761844.1, YP_040853.1, YP_003154888.1, YP_003142045.1, YP_002280953.1, NP_371963.1, NP_422368.1, EGC98966.1, EGC76398.1, YP_004263661.1, YP_004252039.1, YP_679036.1, YP_499973.1, ZP_08054972.1, ZP_08053009.1, ZP_04067276.1, ZP_03968868.1, ZP_03963857.1, ZP_03933079.1, ZP_03497046.1, ZP_06668924.1, ZP_06667106.1, ZP_06324464.1, ZP_06196777.1, ZP_05114159.1, ZP_05083968.1, ZP_05070370.1, ZP_05030022.1, ZP_04673064.1, ZP_03517011.1, ZP_03505783.1, XP_001310698.1, ABK27691.1 oder CAB59281.2 verwendet werden. Für den Fall, dass die Zelle eine Alanindehydrogenase exprimiert, ist es vorteilhaft, eine anorganische Stickstoffquelle, bevorzugt ein Ammoniumsalz wie Ammoniumchlorid oder Ammoniumsulfat, in ausreichender Menge zur wässrigen Lösung zu geben. Ein Verfahren zur Erhöhung der Alaninkonzentration ist in der EP12162846.5 beschrieben.

Ein Aspekt der vorliegenden Erfindung sieht die Verwendung eines Knockouts eines für ein Polypeptid umfassend SEQ ID NO 2 oder eine Variante davon kodierenden Gens als Teil der genetischen Ausstattung einer rekombinanten Zelle zur Erhöhung der Produktion eines Carbonsäureesters der Formel (I) vor. Dies bedeutet, dass der Knock out eines für ein Polypeptid umfassend SEQ ID NO 2 oder eine Variante davon kodierenden Gens als Merkmal der Zelle mit dem Zweck vorgenommen wurde, die Ausbeute, Kohlenstoff- und/oder Stickstoffbilanz und/oder Reinheit des Produktes der Umsetzung des Carbonsäureesters der Formel (I) zu erhöhen.

In einer bevorzugtesten Ausführungsform sieht die Erfindung eine Zelle vor, bei der es sich um eine *E.* coli-Zelle handelt, die einen Knockout des Polypeptides umfassend SEQ ID NO 2 oder einer Variante sowie des Polypeptides FadL im Genom der Zelle aufweist, wobei die Zelle weiterhin eine Alkanhydroxylase, bevorzugt AlkB aus *Pseudomonas putida,* eine Transaminase, bevorzugt die Transaminase von *Chromobacterium violaceum* ATCC 12472, eine Alanindehydrogenase, bevorzugt die Alanindehydrogenase aus *Bacillus subtilis,* und AlkL aus *Pseudomonas putida* exprimiert. Diese Zelle wird in einer weiteren bevorzugtesten Ausführungsform in einer wässrigen Lösung mit einem Fettsäureester, bevorzugt Laurinsäuremethylester, kontaktiert.

## Patentansprüche

1. Verfahren zur Umsetzung eines Carbonsäureesters der Formel (I)
R¹ - A - COOR² (I),
wobei R¹ aus der Gruppe ausgewählt ist, die Wasserstoff, -CH₂OH, -CHO, -COOR³, -CH₂SH, - CH₂OR³ und -CH₂NH₂ umfasst,
wobei R² aus der Gruppe ausgewählt ist, die Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst,
und wobei es sich bei A um einen Alkylrest mit wenigstens vier Kohlenstoffatomen handelt,
mittels einer Zelle, umfassend den Schritt
a) Kontaktieren der Zelle mit dem Carbonsäureester in einer wässrigen Lösung,
wobei es sich bei der Zelle um eine rekombinante Zelle handelt, bei der die Aktivität eines Polypeptides umfassend SEQ ID NO 2 oder eine Variante davon gegenüber dem Wildtyp der Zelle verringert ist.

2. Verfahren nach Anspruch 1, weiter umfassend den Schritt
b) Kontaktieren der wässrigen Lösung mit einer hydrophoben organischen Lösung umfassend einen Kationenaustauscher.

3. Verwendung eines Knockouts eines für ein Polypeptid mit umfassend SEQ ID NO 2 oder eine Variante davon kodierenden Gens als Teil der genetischen Ausstattung einer rekombinanten Zelle zur Erhöhung der Produktion eines Carbonsäureesters der Formel (I)
R¹ - A - COOR² (I),
wobei R¹ aus der Gruppe ausgewählt ist, die Wasserstoff, -CH₂OH, -CHO, -COOR³, -CH₂SH, - CH₂OR³ und -CH₂NH₂ umfasst,
wobei R² aus der Gruppe ausgewählt ist, die Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst,
und wobei es sich bei A um einen Alkylrest mit wenigstens vier Kohlenstoffatomen handelt,
gegenüber dem entsprechenden Wildtyp der Zelle.

4. Verwendung einer rekombinanten Zelle, bei der die Aktivität eines Polypeptides umfassend SEQ ID NO 2 oder eine Variante davon gegenüber dem Wildtyp der Zelle verringert ist, zur Umsetzung eines Carbonsäureesters der Formel (I)
R¹-A-COOR² (I),
wobei R¹ aus der Gruppe ausgewählt ist, die Wasserstoff, -CH₂OH, -CHO, -COOR³, -CH₂SH, - CH₂OR³ und -CH₂NH₂ umfasst,
wobei R² aus der Gruppe ausgewählt ist, die Wasserstoff und Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst,
und wobei es sich bei A um einen Alkylrest mit wenigstens acht Kohlenstoffatomen handelt.

5. Verfahren oder Verwendung nach einem der Ansprüche 1 bis 4, wobei es sich bei A um einen gesättigten Alkylrest handelt, bevorzugt um einen Alkylrest der Formel -(CH₂)ₙ-, wobei n wenigstens 4 ist.

6. Verfahren nach einem der Ansprüche 1 bis 2, wobei R¹ aus der Gruppe ausgewählt ist, die Wasserstoff, -CH₂OH, -CHO und -CH₂NH₂ umfasst.

7. Verfahren nach einem der Ansprüche 1 bis 6, wobei es sich bei A um einen substituierten, nicht substituierten, linearen, verzweigten und/oder zyklischen Alkyl-, Aryl- oder Aralkylrest mit wenigstens 4, bevorzugter 6, noch bevorzugter 8 Kohlenstoffatomen handelt.

8. Zelle, die eine rekombinante Alkanhydroxylase exprimiert, wobei die Aktivität eines Polypeptides umfassend SEQ ID NO 2 oder eine Variante davon gegenüber dem Wildtyp der Zelle verringert ist.

9. Zelle nach Anspruch 7 wobei es sich bei der Alkanhydroxylase um eine Alkanhydroxylase aus der Gruppe umfassend eine AlkB-Monooxygenase und eine Cytochrom P450-Monooxygenase der CYP153-Familie handelt.

10. Reaktionsmischung umfassend die Zelle nach einem der Ansprüche 8 oder 9 in wässriger Lösung sowie einen Carbonsäureester der Formel (I)
R¹ - A - COOR² (I),
wobei R¹ aus der Gruppe ausgewählt ist, die Wasserstoff, -CH₂OH, -CHO, -COOR³, -CH₂SH, - CH₂OR³ und -CH₂NH₂ umfasst,
wobei R² aus der Gruppe ausgewählt ist, die Alkyl, bevorzugt Methyl, Ethyl und Propyl, umfasst,
wobei R³ aus der Gruppe ausgewählt ist, die Wasserstoff und Alkyl, bevorzugt Methyl, Ethyl und Propyl, umfasst,
wobei es sich bei A um einen Alkylrest mit wenigstens vier Kohlenstoffatomen handelt, wobei es sich bei A bevorzugt um einen gesättigten Alkylrest handelt, bevorzugt um einen Alkylrest der Formel -(CH_{z})ₙ-, wobei n wenigstens 4 ist,
wobei R¹ bevorzugt aus der Gruppe ausgewählt ist, die Wasserstoff, -CH₂OH, -CHO und -CH₂NH₂ umfasst,
und wobei es sich bei A bevorzugt um einen substituierten, nicht substituierten, linearen, verzweigten und/oder zyklischen Alkyl-, Aryl- oder Aralkylrest mit wenigstens 4, bevorzugter 6, noch bevorzugter 8 Kohlenstoffatomen handelt.

11. Reaktionsmischung nach Anspruch 10, weiter umfassend eine hydrophoben organischen Lösung mit einem Kationenaustauscher.

12. Verfahren, Zelle, Reaktionsmischung oder Verwendung nach einem der Ansprüche 1 bis 11, wobei die Zelle weiterhin eine Transaminase exprimiert.

13. Verfahren, Zelle, Reaktionsmischung oder Verwendung nach einem der Ansprüche 1 bis 12, wobei die Zelle weiterhin eine Alanindehydrogenase exprimiert.

14. Verfahren, Zelle, Reaktionsmischung oder Verwendung nach einem der Ansprüche 1 bis 13, wobei die Zelle weiterhin ein Protein aus der AlkL-Familie aufweist.

15. Verfahren, Zelle, Reaktionsmischung oder Verwendung nach einem der Ansprüche 1 bis 14, wobei die Zelle eine ihrem Wildtyp gegenüber verringerte Aktivität wenigstens eines Enzyms aufweist, das eine der Reaktionen der β-Oxidation von Fettsäuren katalysiert, wobei es sich bevorzugt um ein Enzym aus der Gruppe handelt, die Fettsäure-CoA-Ligase, Acyl-CoA-Dehydrogenase, 2,4-Dienoyl-CoA-Reduktase, Enoyl-CoA-Hydratase and 3-Ketoacyl-CoA-Thiolase, einen Fettsäureimporter oder Varianten davon umfasst, besonders bevorzugt um FadL oder eine Variante davon.

16. Verfahren, Zelle, Reaktionsmischung oder Verwendung nach einem der Ansprüche 1 bis 15, wobei die Zelle wenigstens ein Enzym aus der Gruppe umfassend Alkanhydroxylase, Alkoholdehydrogenase, Transaminase, Alanindehydrogenase und Protein aus der AlkL-Familie in rekombinanter Form aufweist und/oder überexprimiert.

17. Verfahren, Zelle, Reaktionsmischung oder Verwendung nach einem der Ansprüche 1 bis 16, wobei die Aktivität eines Polypeptides umfassend SEQ ID NO 2 oder eine Variante davon gegenüber dem Wildtyp der Zelle durch Knock out eines für ein Polypeptid umfassend SEQ ID NO 2 oder eine Variante oder einer Variante davon kodierenden Gens verringert ist.
